# EUROPÄISCHE PATENTSCHRIFT

(11) **EP 2 604 202 B1**
(45) Veröffentlichungstag und Bekanntmachung des Hinweises auf die Patenterteilung: **01.04.2015**
(21) Anmeldenummer: 11193542.5
(22) Anmeldetag: 14.12.2011
(51) Int. Cl.: A61B 17/3203, A61B 18/12, A61B 17/00, A61B 18/14, A61B 19/00

(54) **Instrument für die Wasserstrahlchirurgie**
Instrument for water jet surgery
Instrument pour la chirurgie par jet d'eau

(43) Veröffentlichungstag der Anmeldung: 19.06.2013
(73) Patentinhaber: ERBE Elektromedizin GmbH, 72072 Tübingen (DE)
(72) Erfinder: Schmidt, Steffanie, 72124 Pliezhausen (DE); Kühner, Ralf, 70567 Stuttgart (DE)
(74) Vertreter: Rüger, Barthelt & Abel

(56) Entgegenhaltungen:
- WO-A2-2009/009274
- WO-A2-2009/121563
- DE-A1-102009 017 636
- US-A- 5 505 729

## Beschreibung

Die Erfindung betrifft ein chirurgisches Instrument für die Wasserstrahlchirurgie, gegebenenfalls in Kombination mit Hochfrequenzchirurgie.

Aus der EP 0 530 400 B1 ist ein Instrument für die Hochfrequenzchirurgie und zum Schneiden und Koagulieren mit HF-Strom bekannt, wobei dieses Instrument einen flexiblen Schaft aufweist, an dessen Spitze eine einziehbare nadelförmige HF-Elektrode angeordnet ist. Zur mechanischen Bewegung und zur Stromversorgung derselben führt ein Kabel durch den Schaft. Das Kabel dient sowohl der elektrischen Versorgung der Elektrode als auch zur Übertragung einer mechanischen Bewegung auf die Elektrode.

Aus der EP 0 536 440 B1 ist es zusätzlich bekannt, den Schaft als Fluidleitungselement auszubilden, durch das der HF-Elektrode Spülfluide zugeführt werden können und durch das auch eine Absaugung vorgenommen werden kann.

Weiter ist aus der CA 2497897 A1 ein endoskopisches Instrument bekannt, an dessen distalen Ende eine Schere angeordnet ist. Diese wird durch Fluidzylinder betätigt, die über Fluidleitungsmittel mit einem am proximalen Ende vorgesehenen hydraulischen Betätigungsorgan verbunden sind.

Außerdem ist es beispielsweise aus der DE 10 2009 017 636 A1 bekannt, mittels einer Flüssigkeitsstrahleinrichtung, die an ihrem distalen Ende eine Düse aufweist, eine Dissektion und/oder nadellose Injektion in einem Gewebe durchzuführen. Außerdem ist es aus dieser Druckschrift bekannt, eine solche Flüssigkeitsstrahleinrichtung mit einer HF-Elektrode zu versehen, um weitere Behandlungsmaßnahmen, wie bspw. Schneiden und/oder Koagulieren eines Gewebes mittels HF-Strom durchzuführen. So kann der Operateur wahlweise Flüssigkeitsstrahlen oder HF-Strom in das Gewebe einleiten, um den gewünschten Effekt hervorzurufen, wobei er dazu jedoch nicht jedes Mal das Instrument wechseln muss. Dies verkürzt die Operationszeit und vereinfacht die Handhabung des Instruments.

Die WO 2009/121563 offenbart die Merkmale des Oberbegriffs von Anspruch 1 und beschreibt ein Wasserstrahlchirurgieinstrument, an dessen distalen Ende ein Schaft linear beweglich gelagert ist. Dieser ist flexibel ausgebildet und in einem Rohr gelagert, so dass er vorgeschoben und zurückgezogen werden kann. Die Bewegungsübertragung erfolgt rein mechanisch von proximal angeordneten Handgriffen des Instruments.

Die WO 2009/009274 A1 beschreibt ein chirurgisches Instrument mit einer Elektrode, die in einem Sondenschlauch linear beweglich angeordnet ist. Die Bewegung der schlingenförmigen oder anderweitig ausgebildeten elektrischen Sonde erfolgt mechanisch.

Es ist Aufgabe der Erfindung, ein fluidchirurgisches Instrument hinsichtlich seiner Handhabbarkeit weiter zu verbessern.

Diese Aufgabe wird mit dem Instrument nach Anspruch 1 gelöst:
Das erfindungsgemäße Instrument weist einen Instrumentenkopf auf, an dem eine Düse beweglich gelagert ist. Die Düse kann zwischen einer ersten Position und einer zweiten Position hin und her bewegt werden. Bei der Bewegung handelt es sich vorzugsweise um eine Linearbewegung. Es sind jedoch auch andere Bewegungen, z.B. Schwenkbewegungen oder Drehbewegungen, möglich. Bei den beiden Positionen kann es sich um eine Aktivposition und um eine Passivposition handeln. Die Beweglichkeit der Düse zwischen einer Aktivposition und einer Passivposition ermöglicht es, die Düse, beispielsweise beim Positionieren in einer Körperhöhle des Patienten oder im Gewebe des Patienten in eine geschützte Position zu bringen. Die Düse kann deshalb sehr filigran, bspw. als schlanke Nadel, ausgebildet werden. Die Gefahr, dass sie bei einer Positionierung des Instruments im Gewebe verbiegt oder abbricht, ist nicht gegeben, wenn sich diese Düse in geschützter Position, bspw. in einer Rückzugsposition (Passivposition) befindet.

Zur Durchführung einer Behandlung kann die Düse in eine Aktivposition gebracht werden. Beispielsweise kann eine nadelförmige Düse dann längs vorgeschoben werden und liegt somit frei und im Gewebe exponiert. Die Düse kann hier z.B. als HF-Elektrode dienen, um HF-Strom in das Gewebe einzuleiten und einen gewünschten chirurgischen Effekt zu erbringen. Es ist auch möglich, die Düse zum Ausstoßen eines Wasserstrahls zu nutzen. Vorzugsweise geschieht dies ebenfalls in vorgeschobener Position der Düse, d.h. in Aktivposition. Alternativ kann einer Wasserstrahlinjektion jedoch auch bei in rückgezogener Position befindlicher Düse erfolgen. Dies liegt im Rahmen verschiedener Ausführungsmöglichkeiten.

Erfindungsgemäß ist die Düse mit einem hydraulischen Aktor verbunden, durch den die Düse zwischen zumindest zwei Positionen, d.h. zwischen Aktivposition und Passivposition hin und her bewegbar ist. Der hydraulische Aktor weist eine Fluidkammer auf, die mit einem Hydraulikfluid beaufschlagt ist. Die in oder an dem Instrumentenkopf vorgesehene Fluidkammer steht über ein Fluidleitungselement, bspw. ein Rohr oder einen Schlauch oder eine Kombination aus beiden, mit einem Instrumentenanschlusskörper in Verbindung. Von diesem wird durch das Fluidleitungselement Hydraulikfluid zu dem Aktor geleitet, der dadurch die Düse in Aktivposition oder in Passivposition überführt.

Vorzugsweise dient der Aktor dazu, die Düse in Aktivposition zu überführen, während sie z.B. durch ein Federmittel in ihre Rückzugsposition bzw. Passivposition hin vorgespannt sein kann. Dies gilt insbesondere, wenn die Düse linear beweglich gelagert ist. Vorzugsweise ist die Düse linear längs einer von ihr festgelegten Strahlrichtung beweglich gelagert. Ist die Düse nadelförmig, wird sie dabei entlang ihrer Längsrichtung bewegt.

Der hydraulische Aktor kann vorzugsweise als Kolben-Zylinder-Einheit ausgebildet sein. Es sind jedoch auch andere Aktoren möglich, wie bspw. Ballonanordnungen oder dergleichen, die die Änderung eines Flüssigkeitsdrucks oder Flüssigkeitsvolumens in eine mechanische Bewegung umsetzen. Die so gewonnene Antriebsbewegung kann eine Schubbewegung, eine Schwenkbewegung oder eine Zugbewegung sein.

Als Hydraulikmedium ist vorzugsweise eine physiologisch unbedenkliche Flüssigkeit vorgesehen, beispielsweise eine Natriumchloridlösung, vorzugsweise eine physiologische Kochsalzlösung. Es kann sich dabei um das von der Düse auszustoßende Fluid oder auch um ein in gesonderten Kanälen geführtes Fluid handeln. Der Fluiddruck wird mittels einer Stellvorrichtung zur hydraulischen Betätigung des Aktors variiert. Diese Stellvorrichtung kann bspw. handbetätigt oder auch durch andere technische Mittel, wie Pumpen oder dergleichen, gebildet sein. Die Düse kann mit einer Quelle für hochfrequentem Strom verbunden sein. Der hochfrequente Strom (bzw. die hochfrequente Spannung) kann über ein geeignetes elektrisches Leitungsmittel von dem Instrumentenanschlusskörper zu dem Instrumentenkopf und dort insbesondere zu der Düse geführt werden, die dann als Elektrode dient. Gegebenenfalls können jedoch auch gesonderte Elektroden vorgesehen sein. Zur elektrischen Leitung kann eine sich durch das Fluidleitungselement erstreckende isolierte oder nicht isolierte Metalllitze dienen. Es ist jedoch auch möglich, das in dem Fluidleitungselement vorhandene hydraulische Fluid, insbesondere wenn es ein Elektrolyt, bspw. eine NaCl-Lösung, ist, als elektrischen Leiter zu nutzen. Es können damit hoch flexible und sehr schlanke Instrumente für die Wasserstrahlchirurgie und insbesondere für die kombinierte Wasserstrahl-Elektro-Chirurgie aufgebaut werden.

Die Düse weist vorzugsweise einen Düsenkanal auf, der mit der Fluidkammer und somit mit dem (ersten) Fluidleitungselement in Verbindung steht. Es ist jedoch auch möglich ein zweits Fluidleitungselement vorzusehen, das der Düse Fluid zuführt, während das andere (erste) Fluidleitungselement nur der Betätigung des Aktors dient. Die beiden Fluidleitungselemente können zueinander koaxial (eines in dem anderen) oder auch anderweitig, zum Beispiel parallel zueinander angeordnet, sein.

Vorzugsweise ist der Düse ein Ventil zugeordnet, das die Verbindung von dem zuführenden Fluidleitungselement zu der Düse freigeben oder sperren kann. Dieses Ventil kann elektrisch oder hydraulisch betätigt sein. Beispielsweise kann es über die zugeleitete HF-Spannung betätigt werden. Es kann auch mittels einer der HF-Spannung überlagerten Gleichspannung bestätigt sein. Alternativ kann es über eine gesonderte Leitung mit einer Steuerspannung versehen werden. Das Ventil kann außerdem von dem Fluiddruck betätigt werden, der an der Düse und/oder an dem hydraulischen Aktor anliegt.

Weitere Einzelheiten vorteilhafter Ausführungsformen der Erfindung sind Gegenstand der Zeichnung, der Beschreibung oder von Ansprüchen. Es zeigen:
Figur 1 ein kombiniertes Wasserstrahl-HF-Chirurgie-Instrument, in schematischer Längsschnittdarstellung, in einer ersten Ausführungsform, und
Figur 2-4 weitere Ausführungsformen von kombinierten Wasserstrahl-HF-Chirurgie-Instrumenten, jeweils in längs geschnittener schematischer Darstellung.

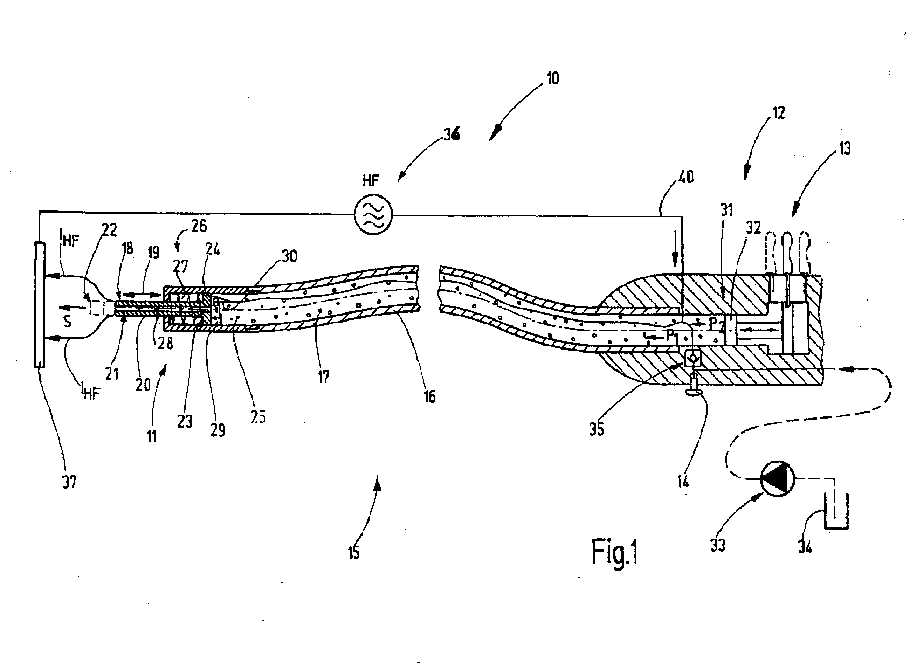

In Figur 1 ist ein Instrument 10 schematisch veranschaulicht, das sich mindestens zur Durchführung von wasserstrahlchirurgischen Eingriffen eignet. Das Instrument 10 beinhaltet einen Instrumentenkopf 11, der das vom Operateur aus gesehen distale Ende des Instruments 10 bildet. Das proximale Ende des Instruments 10 wird durch einen Instrumentenanschlusskörper 12 gebildet, der Betätigungselemente 13, 14 zur Aktivierung oder Deaktivierung des Instruments 10 enthalten kann. Der Instrumentenanschlusskörper 12 kann als Handgriff oder anderweitig ausgebildet sein.

Zwischen dem Instrumentenanschlusskörper 12, der das proximale Ende des Instruments 10 bildet, und dem Instrumentenkopf 11 ist ein Fluidleitungselement 15 angeordnet. Dieses wird beispielsweise durch einen flexiblen Schlauch 16, ein starres oder biegsames Rohr oder eine Kombination aus Schlauch- und Rohrabschnitten oder ein sonstiges Mittel gebildet, das einen Kanal 17 umschließt, in dem sich eine Flüssigkeit befinden kann. Die Flüssigkeit bildet ein Hydraulikfluid. Im vorliegenden Fall ist die Flüssigkeit vorzugsweise eine Natriumchloridlösung z.B. eine physiologische Kochsalzlösung. An dem Instrumentenkopf 11 ist eine Düse 18 vorgesehen, die, wie ein Pfeil 19 andeutet, längsbeweglich gelagert ist.

Zu der Düse 18 gehört vorzugsweise ein längliches nadelartiges Rohr 20, an dessen Ende ein Düsenkörper 21 angeordnet sein kann. Die Düse 18 ist vorzugsweise darauf eingerichtet einen dünnen Fluidstrahl auszustoßen, der beispielsweise Gewebeschichten voneinander Trennen oder perforieren und somit Fluid in das Gewebe einleiten kann. Bei dem Fluidstrahl kann es sich um einen laminaren oder einen turbulenten, z.B. kegelförmigen oder anders geformten Fluidstrahl handeln. Vorzugsweise besteht das Rohr 20 aus einem Metall.

Die Düse 18 ist zwischen einer zurückgezogenen inaktiven Position und einer vorgeschobenen Aktivposition längs einer Strahlrichtung S bewegbar. Figur 1 veranschaulicht die Rückzugsposition, in der die Düse 18 wenigstens teilweise in den Instrumentenkopf 11 eingefahren ist. Die Düse 18 kann axial aus dem Instrumentenkopf 11 herausgefahren werden. Eine vorgeschobene Aktivposition ist in Figur 1 durch gestrichelte Linien 22 markiert.

Zur Bewegung der Düse 18 ist in dem Instrumentenkopf 11 ein hydraulischer Aktor 23 vorgesehen. Dieser wird beispielsweise durch einen z.B. aus Metall bestehenden Kolben 24 gebildet, der in wenigstens eine Seite einer Fluidkammer 25 grenzt. Die Fluidkammer 25 besteht vorzugsweise aus einem elektrisch isolierenden Material, vorzugsweise Kunststoff, oder ist anderweitig nach außen hin isoliert.

In diesem Ausführungsbeispiel ist die Fluidkammer 25, beispielsweise eine Zylinderkammer z.B. mit Kreisquerschnitt. Der in dieser Fluidkammer 25 abgedichtet sitzende, bewegliche Kolben 24 ist vorzugsweise starr mit der Düse 18 bzw. dem Rohr 20 verbunden, das insoweit als Kolbenstange angesehen werden kann. Optional ist dem Kolben 24 ein Federmittel 26, z.B. in Gestalt einer Schraubenfeder 27 zugeordnet, die den Kolben 24 und somit die Düse 18 in eine Rückzugs- oder Passivposition hin vorspannt. Durch die Düse 18 bzw. das Rohr 20 sowie durch den Kolben 24 erstreckt sich ein Düsenkanal 28, über den von der Düse 18 auszuspritzende Fluid zu der Düsenmündung gelangt.

Optional, jedoch vorzugsweise, ist der Düse 18 ein gesteuertes Ventil 29 zugeordnet. Dieses weist zumindest zwei Ventilstellungen, nämlich Verschluss und Freigabe auf. Das Ventil 29 ist zwischen dem Düsenkanal 28 und dem Kanal 17 angeordnet. Im vorliegenden Ausführungsbeispiel wird es über eine elektrische Leitung 30 gesteuert. Diese erstreckt sich durch den Kanal 17 von dem Instrumentenkopf 11 zu dem Instrumentenanschlusskörper 12 hin. Die Leitung 30 kann eine flexible isolierte Litze sein. Es kann sich bei der Leitung 30 auch um eine nicht isolierte flexible elektrische Leitung handeln, die dann mit dem in dem Kanal 17 befindlichen Elektrolyt in elektrischem Kontakt steht.

Der Instrumentenanschlusskörper 12 enthält eine Stellvorrichtung 31 die in Form einer Pumpenvorrichtung ausgebildet sein kann, die vorzugsweise dazu eingerichtet ist, bei Betätigung eine vorgegebene Fluidmenge zu verdrängen und durch den Kanal 17 zu dem Instrumentenkopf 11 zu fördern. Diese Stellvorrichtung 31 wird beispielsweise durch einen in eine Bohrung verschiebbar gelagerten Kolben 32 gebildet, über den ein Druck P₂ auf die in dem Kanal 17 stehende Flüssigkeitssäule ausgeübt werden kann. Zum Verschieben des Kolbens 32 kann z.B. das Betätigungselement 13 dienen, das der Operateur von Hand betätigen kann. Es können jedoch auch andere Betätigungseinrichtungen, wie Zugmagnete oder dergleichen, vorgesehen sein.

Das Instrument 10 ist an ein Gerät angeschlossen, das in Figur 1 nicht weiter veranschaulicht ist. Zu dem Gerät gehört eine in Figur 1 schematisch dargestellte Vorrichtung beispielsweise in Form einer Pumpvorrichtung 33, mittels derer das der Düse 18 zuzuleitende Fluid, bspw. NaCl-Lösung, aus einem Vorrat 34 zunächst zu dem Instrumentenanschlusskörper 12 und von diesem über das Fluidleitungselement 15 zu dem Instrumentenkopf 11 und somit zu der Düse 18 geleitet wird. Zur Steuerung des Fluidzuflusses zu dem oder in den Kanal 17 kann ein Ventil vorgesehen sein, das über das Betätigungselement 14 z.B. von Hand freigegeben werden kann. Die Pumpvorrichtung 33 fördert vorzugsweise mit einem Druck P₁, der zur Erzeugung des aus der Düse 18 austretenden Fluidstrahls dient.

Das Ventil 14 kann über die Leitung 30 mit dem Ventil 29 verbunden sein, um das Ventil 29 zu steuern. Auf diese Weise kann das Ventil 29 immer dann geöffnet werden, wenn über das Betätigungselement 14 das entsprechende Fluid mit Druck in den Kanal 17 gegeben wird.

Optional kann das Instrument 10 außerdem als HF-chirurgisches Instrument dienen und dazu an eine HF-Stromquelle bzw. HF-Spannungsquelle angeschlossen sein. Diese HF-Spannungsquelle 36 kann Teil eines Geräts das nicht weiter veranschaulicht ist sein, welches das Instrument 10 mit HF-Leistung versorgt. Die HF-Spannungsquelle 36 ist einerseits an eine Neutralelektrode 37 angeschlossen, die vorzugsweise großflächig mit dem Patienten verbunden ist, und andererseits mit dem Instrumentenanschlusskörper 12 verbunden, über den die HF-Spannung an den Instrumentenkopf 11 geleitet wird. Dazu kann wiederum die Leitung 30 dienen, die sich durch das Fluidleitungselement 15 erstreckt. Alternativ kann die Leitung 30 auch in die Wand des Fluidleitungselements 15 eingebettet sein.

Im chirurgischen Einsatz wird das Instrument 10 mindestens mit seinem Instrumentenkopf 11 und teilweise mit dem Fluidleitungselement 15 in eine Körperhöhle oder das Gewebe eines Patienten eingeführt. Dies kann endoskopisch, laproskopisch oder auch bei offenem Operationsgebiet geschehen. Beim Einführen des Instruments 10 befindet sich die Düse 18 in einer rückgezogenen Position, in der das dünne nadelartige Rohr 20 geschützt wenigstens teilweise im Inneren des Instrumentenkopfs 11 liegt.

Soll die Düse 18 aktiviert werden, kann sie in eine vorgeschobene Position bewegt werden. Dazu betätigt der Chirurg das Betätigungselement 13. Er schiebt damit den Kolben 32 vor, um Fluid zu verdrängen. Mit anderen Worten, die Stellvorrichtung 31 (oder eine sonstige entsprechende Pumpvorrichtung) wird aktiviert und schiebt somit über die Kopplung der in dem Kanal 17 vorhandenen Flüssigkeitssäule den Kolben 24 gegen die Kraft des Federmittels 26 in distaler Richtung. Die insoweit vorgeschobene Düse 18 kann nunmehr elektrisch oder auch fluidtechnisch aktiviert werden.

Zur elektrischen Aktivierung wird der HF-Generator 36 bspw. durch einen Fußschalter, Handschalter oder dergleichen eingeschaltet, so dass die Düse 18 an ihrem Außenmantel oder an Teilen desselben und/oder an ihrer Stirnfläche HF-Strom in das Gewebe gibt. Dieser ist in Figur 1 links durch divergierende Pfeile I_{HF} angedeutet.

Zur fluidtechnischen Aktivierung wird das Fluidleitungsmittel 14 aktiviert. Es wird dadurch zusätzliches Elektrolyt, bspw. NaCl-Lösung in den Kanal 17 gefördert. Zugleich wird über die Leitung 30 das Ventil 29 geöffnet, so dass ein Fluidstrahl aus der Düse 18 austreten und die gewünschte Wirkung hervorrufen kann. Der Operateur muss gleichzeitig das Betätigungselement 13 in vorgeschobener Position festhalten oder in der vorgeschobenen Position arretieren.

Die Bestätigung des Ventils 29 kann wahlweise von verschiedenen Ereignissen abhängig gemacht und über verschiedene Mechanismen gesteuert werden. Je nach Ausführungsform kann die Leitung 30 bspw. mehradrig ausgebildet sein. Eine erste Ader dient dann der Betätigung des Ventils 29 bspw. in Abhängigkeit von der Betätigung des Elements 14 oder dem Öffnen des Rückschlagventils 35, das insoweit als Schalter dienen kann. Alternativ kann das Ventil 29 über ein gesondertes Betätigungselement gesteuert werden.

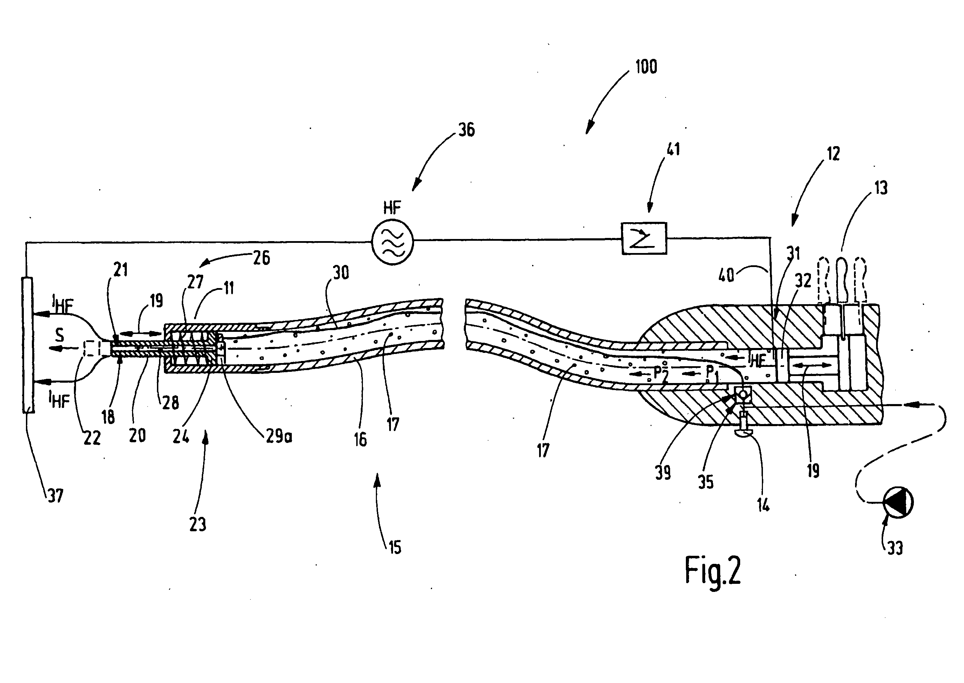

Figur 2 veranschaulicht eine abgewandelte Ausführungsform des erfindungsgemäßen Instruments 100. Soweit strukturell oder funktionell gleiche oder ähnliche Elemente vorhanden sind, wird unter Zugrundelegung gleicher Bezugszeichen auf die vorige Beschreibung verwiesen. Im Unterschied zu der vorstehend beschriebenen Ausführungsform dient die Leitung 30 hier lediglich der Steuerung des Ventils 29. Die Leitung 30 kann somit einadrig ausgeführt sein. Sie wird beispielsweise von einem Fluiddruckschalter 39 gesteuert, der dazu dient, zu erfassen, ob der in dem Kanal 17 vorhandene Fluiddruck einen festgelegten Grenzdruck überschreitet. In Figur 2 ist der Fluiddruckschalter 39 symbolisch mit dem Rückschlagventil 35 vereinigt. Alternativ kann ein gesonderter Schalter vorgesehen sein, der auf Betätigung des Betätigungselements 14 anspricht.

In dem Ausführungsbeispiel nach Figur 2 dient das in dem Kanal 17 vorhandene elektrisch leitende Fluid dazu, der Düse 18 HF-Strom zuzuführen. Das Hydraulikfluid ist vorzugsweise eine Kochsalzlösung, die ausreichend elektrisch leitfähig ist. Sie steht an dem Instrumentenanschlusskörper 12 mit einer HF-Strom zuführenden Leitung 40 und an dem Instrumentenkopf 11 mit dem Kolben 24 und somit mit der Düse 18 in elektrischer Verbindung. Außerdem zeigt Figur 2 einen Schalter 41 zur Steuerung der HF-Spannungsquelle 36. Der Schalter 41 ist symbolisch als Schalter in der Leitung 40 eingetragen. Alternativ kann ein solcher Schalter jedoch auch die HF-Quelle 36 direkt steuern.

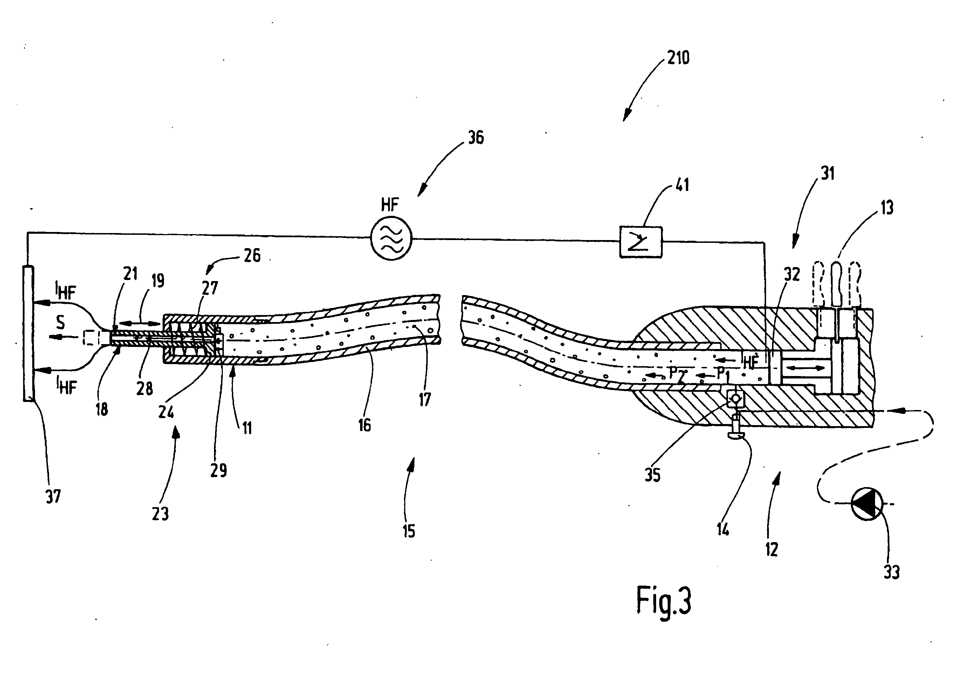

Eine weitere abgewandelte Ausführungsform des Instruments 210 veranschaulicht Figur 3. Soweit deren Elemente bau- und/oder funktionsgleich mit Elementen der vorbeschriebenen Ausführungsformen sind, wird unter Zugrundelegung der bereits eingeführten Bezugszeichen auf die vorige Beschreibung verweisen. Die Besonderheit des Instruments 210 besteht in der Betätigung des Ventils 29 durch den in dem Kanal 17 vorhandenen hydraulischen Druck. Die HF-Zuführung zu dem Kolben 24 und somit zu der Düse 18 kann, wie dargestellt, durch das Elektrolyt (entsprechend Figur 2) oder auch über eine nicht dargestellte Leitung (entsprechend Figur 1) erfolgen. Das Ventil 29 ist so ausgebildet, dass es die Verbindung zwischen dem Kanal 17 und dem Düsenkanal 28 freigibt, wenn der hydraulische Druck in dem Kanal 17 einen Schwellwert übersteigt. Aktiviert der Operateur das Instrument 210 durch Betätigung des Betätigungselements 14, fördert die Pumpvorrichtung 33 Fluid mit hohem Druck in den Kanal 17 und öffnet, sobald sich ein ausreichender Druck aufgebaut hat, das Ventil 29, womit ein scharfer Strahl in Richtung S aus der Düse 18 austritt. Dabei bleibt die Düse 18 in vorgeschobener aktivierter Position. Nach Schließen des Ventils 35, bspw. wenn der Bediener das Betätigungselement 14 loslässt, schließt das Ventil 29, wobei jedoch die Düse 18 so lange vorgeschoben bleibt, wie der Kolben 32 in vorgeschobener Position gehalten wird. Gibt der Operateur oder Bediener das Betätigungselement 13 frei, drückt die Feder 27 den Kolben 24 in Rückzugsposition zurück. Damit kehrt auch der Kolben 32 in seine in Figur 3 rechte Rückzugsposition zurück.

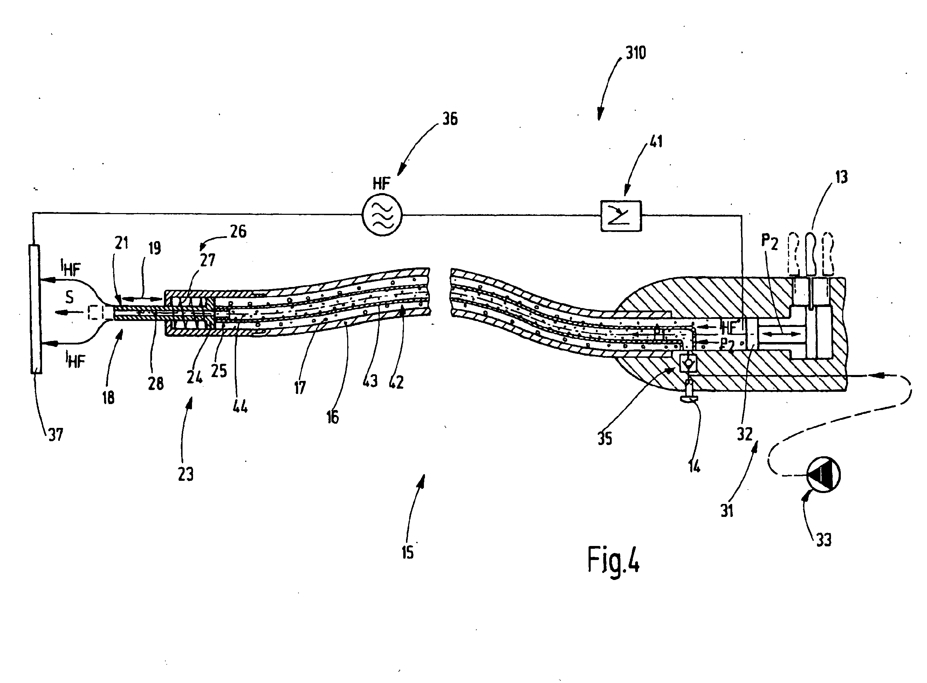

Eine weitere mögliche Abwandlung des erfindungsgemäßen Instruments ist als Instrument 310 in Figur 4 veranschaulicht. Dieses Instrument 310 kommt ohne das Ventil 29 aus. Es weist zusätzlich zu dem ersten Fluidleitungselement 15 ein zweites Fluidleitungselement 42 auf, das bspw. als Schlauch 43 ausgebildet ist. Das Fluidleitungselement 42 kann sich durch den Kanal 17 des ersten Fluidleitungselements 15 erstrecken. Das Fluidleitungselement 42 kann an den Kolben 24 oder einen Fortsatz desselben angeschlossen sein. Der innere Kanal 44 des Fluidleitungselements 42 kommuniziert somit mit dem Düsenkanal 28. Das proximale Ende des Fluidleitungselements 42 kann über ein von dem Betätigungselement 14 gesteuertes Ventil und zum Beispiel über das Rückschlagventil 35 mit der Pumpvorrichtung 33 verbunden sein. In dieser Ausführungsform ist das Rückschlagventil 35 optional. Während der Kanal 17 hier lediglich der Betätigung des Kolbens 24 oder eines sonstigen Aktors 23 dient, ist der Kanal 44 zur Speisung der Düse 18 vorgesehen. Die (optionale) Zuleitung von HF-Strom zu der Düse 18 kann, wie in Figur 4 dargestellt, über das leitfähige Elektrolyt in dem Kanal 17 oder auch z.B. entsprechend Figur 1 über eine gesonderte elektrische Leitung erfolgen. Diese kann wiederum in der Wandung des Schlauchs 16 angeordnet sein oder sich durch den inneren Kanal 17 erstrecken. Es ist auch möglich, die nicht weiter veranschaulichte Leitung mit dem inneren Schlauch 43 zu vereinen. Ansonsten gilt die vorige Beschreibung entsprechend.

Bei einem chirurgischen Instrument 10, 110, 210, 310, das wenigstens zur Durchführung wasserstrahlchirurgischer Eingriffe dient, ist eine Düse 18 vorgesehen, die durch einen hydraulischen Aktor 23 aus einer Rückzugs- oder Passivposition in eine vorgeschobene Aktivposition bewegt werden kann. Die Betätigung erfolgt durch Hydraulikfluid, das dem Instrumentenkopf 11 über ein Rohr 20 und/oder einen Schlauch 43 zugeführt wird. Als Hydraulikfluid kann das der Düse 18 zugeführte, in das Gewebe zu injizierende Fluid, bspw. Natriumchloridlösung, genutzt werden. Vorzugsweise ist die Düse 18 außerdem mit HF-Strom beaufschlagbar. Dieser kann über eine sich durch das Fluidleitungselement 15 erstreckende Leitung oder durch das darin befindliche Elektrolyt zu der Düse geleitet werden.

### Bezugszeichenliste:

- 10, 100, 210, 310: Instrument
- 11: Instrumentenkopf
- 12: Anschluss
- 13, 14: Betätigungselemente
- 15: erstes Fluidleitungselement
- 16: Schlauch
- 17: Kanal
- 18: Düse
- 19: Pfeil
- 20: Rohr
- 21: Düsenkörper
- 22: gestrichelte Linien - Aktivposition der Düse 18
- 23: Aktor
- 24: Kolben
- 25: Fluidkammer
- 26: Federmittel
- 27: Schraubenfeder
- 28: Düsenkanal
- 29, 29a: Ventil
- 30: Leitung
- 31: Stellvorrichtung
- 32: Kolben
- 33: Pumpvorrichtung
- 34: Vorrat
- 35: Rückschlagventil
- 36: HF-Spannungsquelle
- 37: Neutralelektrode
- 39: Druckschalter
- 40: Leitung
- 41: Schalter
- 42: zweites Fluidleitungselement
- 43: Schlauch
- 44: Kanal von 42
- S: Strahlrichtung

## Patentansprüche

1. Chirurgisches Instrument (10, 100, 210, 310) für die Wasserstrahlchirurgie, kombiniert mit einem Instrumentenanschlusskörper (12),
mit einem Instrumentenkopf (11), in dem eine Düse (18) zwischen zumindest zwei Positionen hin und her beweglich gelagert ist,
mit einem ersten Fluidleitungselement (15), über das der Instrumentenkopf (11) mit dem Instrumentenanschlusskörper (12) verbunden ist, mit
einem Aktor (23), der in dem Instrumentenkopf (11) angeordnet und mit der Düse (18) verbunden ist und der eine Fluidkammer (25) aufweist,
wobei das erste Fluidleitungselement (15) mit der Fluidkammer (25) in Fluidverbindung steht,
wobei in oder an dem Instrumentenanschlusskörper (12) eine Stellvorrichtung (31) vorgesehen ist,
**gekennzeichnet dadurch**, dass die Stellvorrichtung zur hydraulischen Betätigung des Aktors (23) vorgesehen ist, so dass die Düse durch dem Aktor zwischen den zumindest zwei Positionen hin und her bewegbar ist.

2. Instrument nach Anspruch 1, **dadurch gekennzeichnet, dass** die Düse (18) linear längs einer von ihr festgelegten Strahlrichtung (S) beweglich gelagert ist.

3. Instrument nach einem der vorstehenden Ansprüche, **dadurch gekennzeichnet, dass** der Aktor (23) und/oder die Düse (18) durch ein Federmittel (26) auf eine Rückzugsposition hin vorgespannt ist.

4. Instrument nach Anspruch 1, **dadurch gekennzeichnet, dass** die Stellvorrichtung (31) eine Pumpe ist.

5. Instrument nach einem der vorstehenden Ansprüche, **dadurch gekennzeichnet, dass** die Düse (18) mit einer Quelle (36) für hochfrequenten Strom in Verbindung steht.

6. Instrument nach einem der vorstehenden Ansprüche, **dadurch gekennzeichnet, dass** sich eine elektrische Leitung (30) von dem Instrumentenanschlusskörper (12) zu der Düse (18) erstreckt.

7. Instrument nach Anspruch 6, **dadurch gekennzeichnet**, **dass** die elektrische Leitung (30) ein in dem ersten Fluidleitungselement (15) vorhandenes Elektrolyt ist.

8. Instrument nach einem der vorstehenden Ansprüche, **dadurch gekennzeichnet, dass** die Düse (18) einen Düsenkanal (28) aufweist und dass die Fluidkammer (25) mit dem Düsenkanal (28) in Fluidverbindung steht.

9. Instrument nach einem der vorstehenden Ansprüche, **dadurch gekennzeichnet, dass** die Düse (18) einen Düsenkanal (28) aufweist, der mit einem zweiten Fluidleitungselement (42) in Fluidverbindung steht.

10. Instrument nach einem der vorstehenden Ansprüche, **dadurch gekennzeichnet, dass** das erste Fluidleitungselement (15) mit einer Pumpvorrichtung (33) für unter Druck stehendes Fluid in Verbindung steht.

11. Instrument nach Anspruch 9, **dadurch gekennzeichnet**, **dass** das zweite Fluidleitungselement (42) mit einer Pumpvorrichtung (33) für unter Druck stehendes Fluid in Verbindung steht.

12. Instrument nach einem der vorstehenden Ansprüche, **dadurch gekennzeichnet, dass** die Düse (18) mit einem in dem Instrumentenkopf (11) angeordneten Ventil (29) verbunden ist.

13. Instrument nach Anspruch 12, **dadurch gekennzeichnet**, **dass** das Ventil (29) elektrisch steuerbar ist.

14. Instrument nach Anspruch 12, **dadurch gekennzeichnet**, **dass** das Ventil (29) hydraulisch steuerbar ist.

## Claims

1. Surgical instrument (10, 100, 210, 310) for water jet surgery combined with an instrument connection body (12),
with an instrument head (11), in which a nozzle (18) is arranged to be movable back and forth between at least two positions,
with a first fluid delivery element (15), by means of which the instrument head (11) is connected to the instrument connection body (12), with
an actuator (23), which is arranged in the instrument head (11) and is connected to the nozzle (18) and which has a fluid chamber (25),
wherein the first fluid delivery element (15) has a fluid connection to the fluid chamber (25),
wherein a regulating device (31) is provided in or on the instrument connection body (12), **characterised in that** the regulating device is provided for hydraulic operation of the actuator (23) so that the nozzle is movable back and forth between the at least two positions by the actuator.

2. Instrument according to claim 1, **characterised in that** the nozzle (18) is arranged to be movable linearly along a jet direction (S) determined by said nozzle.

3. Instrument according to one of the preceding claims, **characterised in that** the actuator (23) and/or the nozzle (18) is prestressed towards a retraction position by a spring element (26).

4. Instrument according to claim 1, **characterised in that** the regulating device (31) is a pump.

5. Instrument according to one of the preceding claims, **characterised in that** the nozzle (18) connects to a source (36) for high-frequency current.

6. Instrument according to one of the preceding claims, **characterised in that** an electrical lead (30) extends from the instrument connection body (12) to the nozzle (18).

7. Instrument according to claim 6, **characterised in that** the electrical lead (30) is an electrolyte present in the first fluid delivery element (15).

8. Instrument according to one of the preceding claims, **characterised in that** the nozzle (18) has a nozzle duct (28) and that the fluid chamber (25) has a fluid connection to the nozzle duct (28).

9. Instrument according to one of the preceding claims, **characterised in that** the nozzle (18) has a nozzle duct (28), which has a fluid connection to a second fluid delivery element (42).

10. Instrument according to one of the preceding claims, **characterised in that** the first fluid delivery element (15) connects to a pump device (33) for pressurised fluid.

11. Instrument according to claim 9, **characterised in that** the second fluid delivery element (42) connects to a pump device (33) for pressurised fluid.

12. Instrument according to one of the preceding claims, **characterised in that** the nozzle (18) is connected to a valve (29) arranged in the instrument head (11).

13. Instrument according to claim 12, **characterised in that** the valve (29) is electrically controllable.

14. Instrument according to claim 12, **characterised in that** the valve (29) is hydraulically controllable.

## Revendications

1. Instrument chirurgical (10, 100, 210, 310) pour la chirurgie à jet d''eau, combiné à un corps de raccordement d''instrument (12), comprenant une tête d''instrument (11) dans laquelle une buse (18) est montée mobile en va-et-vient entre au moins deux positions, comprenant un premier élément de conduite de fluide (15) par lequel la tête d''instrument (11) est reliée au corps de raccordement d''instrument (12), avec un actionneur (23) qui est disposé dans la tête d''instrument (11) et relié à la buse (18) et qui présente une chambre à fluide (25), le premier élément de conduite de fluide (15) étant en liaison fluidique avec la chambre à fluide (25), un dispositif de positionnement (31) étant prévu dans ou sur le corps de raccordement d''instrument (12), **caractérisé en ce que** le dispositif de positionnement est prévu pour l''actionnement hydraulique de l''actionneur (23), de sorte que la buse peut être déplacée en va-et-vient entre lesdites au moins deux positions par l''actionneur.

2. Instrument selon la revendication 1, **caractérisé en ce que** la buse (18) est montée mobile linéairement suivant une direction de jet (S) définie par elle.

3. Instrument selon l''une des revendications précédentes, **caractérisé en ce que** l''actionneur (23) et/ou la buse (18) sont précontraints par un moyen élastique (26) vers une position rétractée.

4. Instrument selon la revendication 1, **caractérisé en ce que** le dispositif de positionnement (31) est une pompe.

5. Instrument selon l''une des revendications précédentes, **caractérisé en ce que** la buse (18) est reliée à une source (36) de courant à haute fréquence.

6. Instrument selon l''une des revendications précédentes, **caractérisé en ce qu'**'une ligne électrique (30) s''étend du corps de raccordement d''instrument (12) à la buse (18).

7. Instrument selon la revendication 6, **caractérisé en ce que** la ligne électrique (30) est un électrolyte présent dans le premier élément de conduite de fluide (15).

8. Instrument selon l''une des revendications précédentes, **caractérisé en ce que** la buse (18) présente un canal de buse (28) et **en ce que** la chambre à fluide (25) est en liaison fluidique avec le canal de buse (28).

9. Instrument selon l''une des revendications précédentes, **caractérisé en ce que** la buse (18) présente un canal de buse (28) qui est en liaison fluidique avec un deuxième élément de conduite de fluide (42).

10. Instrument selon l''une des revendications précédentes, **caractérisé en ce que** le premier élément de conduite de fluide (15) est en communication avec un dispositif de pompage (33) de fluide sous pression.

11. Instrument selon la revendication 9, **caractérisé en ce que** le deuxième élément de conduite de fluide (42) est en communication avec un dispositif de pompage (33) de fluide sous pression.

12. Instrument selon l''une des revendications précédentes, **caractérisé en ce que** la buse (18) est reliée à une vanne (29) disposée dans la tête d''instrument (11).

13. Instrument selon la revendication 12, **caractérisé en ce que** la vanne (29) est à commande électrique.

14. Instrument selon la revendication 12, **caractérisé en ce que** la vanne (29) est à commande hydraulique.
